# EUROPEAN PATENT APPLICATION

(11) **EP 4 190 808 A1**
(43) Date of publication of application: **07.06.2023**
(21) Application number: 21857418.4
(22) Date of filing: 13.07.2021
(51) Int. Cl.: C07K 16/00, C07K 19/00, C12N 15/62, A61K 35/17, A61K 38/17

(54) **HUMANIZED ANTIBODY, CHIMERIC ANTIGEN RECEPTOR, NUCLEIC ACID, VECTOR, CELL AND USE**

(30) Priority: 19.08.2020 CN 202010836034
(71) Applicant: Suzhou Immunofoco Biotechnology Co., Ltd., Suzhou, Jiangsu 201210 (CN)
(72) Inventor: SHEN, Qingshan, Hefei, Anhui 201203 (CN); XING, Kaixuan, Shanghai 201203 (CN)
(74) Representative: Gramm, Lins & Partner Patent- und Rechtsanwälte PartGmbB
(86) International application number: PCT/CN2021/106097
(87) International publication number: WO 2022/037323

(57) **Abstract**

Provided are a humanized antibody specifically binding to EpCAM, and a chimeric antigen receptor, a nucleic acid, a vector, a cell, and the use associated with the humanized antibody. The humanized antibody comprises a heavy chain variable region and a light chain variable region. The sequence of the heavy chain variable region comprises a sequence shown in SEQ ID.1, a sequence shown in SEQ ID.2 and a sequence shown in SEQ ID.3. The sequence of the light chain variable region comprises a sequence shown in SEQ ID.4, a sequence shown in SEQ ID.5, and a sequence shown in SEQ ID.6. After the humanized antibody is constructed as a chimeric antigen receptor-T Cell, EpCAM-positive tumor cells can be specifically identified and killed, and tumors can be cleared in an animal bod

## Description

### TECHNICAL FIELD

The present disclosure relates to the technical field of antibodies, in particular to humanized antibodies, chimeric antigen receptors, nucleic acids, vectors, cells and uses thereof.

### BACKGROUND

The key to anti-tumor immunotherapy is that the selected antibodies can selectively recognize antigens that are highly expressed at tumor sites. Epithelial cell adhesion molecule (EpCAM) is a major surface antigen associated with human epithelial tumors such as esophageal cancer, gastric cancer, colorectal cancer, prostate cancer, lung cancer, breast cancer, ovarian cancer, renal cancer, hepatocellular and pancreatic cancer, can be highly expressed in various human epithelial tumor cells and progenitor cells and stem cells thereof, and thus has the potential to be an important target for anti-tumor immunotherapy.

So far, mouse-derived antibodies are still an important source for immunotherapy drugs, but mouse-derived antibodies are immunogenic to humans and can be easily eliminated as heterologous proteins in human bodies, thereby weakening their therapeutic effectiveness. Accordingly, mouse-derived antibodies need to be humanized to avoid recognition by the human immune system and avoid the induction of human anti-mouse antibody (HAMA) responses.

Therefore, it is necessary to design a humanized antibody that specifically binds to EpCAM and to provide uses of the humanized antibody so as to avoid the above-mentioned problems in the prior art.

### SUMMARY OF THE INVENTION

The object of the present disclosure is to provide a humanized antibody, a chimeric antigen receptor comprising the humanized antibody, a nucleic acid encoding the humanized antibody or the chimeric antigen receptor, and a vector and a cell comprising the nucleic acid. The antibody, the chimeric antigen receptor, the nucleic acid, the vector and the cell can be used to prepare a medication for treating human EpCAM-positive tumors and a reagent for detecting human EpCAM-positive tumors.

In order to achieve the above purpose, the humanized antibody of the present disclosure comprises a heavy chain variable region and a light chain variable region, and a sequence of the heavy chain variable region comprises a sequence shown in SEQ ID NO: 1, a sequence shown in SEQ ID NO: 2 and a sequence shown in SEQ ID NO: 3, respectively as a sequence of a first heavy chain complementarity determining region, a sequence of a second heavy chain complementarity determining region and a sequence of a third heavy chain complementarity determining region; and a sequence of the light chain variable region comprises a sequence shown in SEQ ID NO: 4, a sequence shown in SEQ ID NO: 5 and a sequence shown in SEQ ID NO: 6, respectively as a sequence of a first light chain complementarity determining region, a sequence of a second light chain complementarity determining region and a sequence of a third light chain complementarity determining region.

In some preferred embodiments, the sequence of the heavy chain variable region is at least 90% identical to any one of SEQ ID NO: 7, SEQ ID NO: 8, SEQ ID NO: 9, SEQ ID NO: 10 and SEQ ID NO: 11; the sequence of the light chain variable region is at least 90% identical to any one of SEQ ID NO: 12, SEQ ID NO: 13, SEQ ID NO: 14 and SEQ ID NO: 15.

In some preferred embodiments, the antibody that specifically recognizes EpCAM can be: a single chain antibody (scFV), a monoclonal antibody, a domain antibody, a Fab fragment, an Fd fragment, an Fv fragment, a F(ab')2 fragment and a derivative thereof, or other forms of antibodies.

In some preferred embodiments, the antibody that specifically recognizes EpCAM can be a humanized antibody.

In some preferred embodiments, the humanized antibody is a full-length antibody having a subtype of any one of hIgG1, hIgG2, hIgG3 and hIgG4.

In some embodiments, a first heavy chain framework region, a second heavy chain framework region and a third heavy chain framework region are connected between the first heavy chain complementarity determining region and the second heavy chain complementarity determining region, between the second heavy chain complementarity determining region and the third heavy chain complementarity determining region, and at the third heavy chain complementarity determining region, respectively.

In some embodiments, a first light chain framework region, a second light chain framework region and a third light chain framework region are connected between the first light chain complementarity determining region and the second light chain complementarity determining region, between the second light chain complementarity determining region and the third light chain complementarity determining region, and at the third light chain complementarity determining region, respectively.

The nucleic acid of the present disclosure comprises a sequence encoding the humanized antibody or a fragment of the sequence, and a nucleotide sequence of the chimeric antigen receptor or a fragment of the sequence.

The vector of the present disclosure comprises the nucleic acid.

The cell of the present disclosure comprises the nucleic acid or the vector.

The present disclosure provides use of any one of the humanized antibody, the chimeric antigen receptor, the nucleic acid, the vector and the cell in the manufacture of a reagent for detecting human tumors.

The present disclosure provides use of any one of the humanized antibody, the chimeric antigen receptor, the nucleic acid, the vector and the cell in the manufacture of a medication for treating human tumors. For example, uses in the manufacture of medications such as an anti-human EpCAM monoclonal antibody, a bispecific antibody, a multispecific antibody, and an antibody-drug conjugate (ADC).

An extracellular antigen binding region of the chimeric antigen receptor of the present disclosure comprises the humanized antibody or a fragment of the antibody.

Preferably, the chimeric antigen receptor further comprises a signal peptide having a sequence derived from at least one of GM-CSF, CD8α and CD28.

Preferably, the chimeric antigen receptor further comprises a hinge region having a sequence derived from at least one of CD8α, CD28, 4-1 BB, ICOS, OX40, CD40, CD80 and IgG.

Preferably, the chimeric antigen receptor further comprises a transmembrane region having a sequence derived from at least one of CD8α, CD28, 4-1 BB, ICOS, OX40, CD40, CD80 and CD3.

Preferably, the chimeric antigen receptor further comprises an intracellular signal region having a sequence derived from a Toll-like receptor, and at least one of CD2, CD27, LFA-1, CD8α, CD28, 4-1BB, ICOS, OX40, CD40, CD80, DAP10, DAP12, CD3ζ, and CD3ε.

In some embodiments, the sequence of the intracellular signal region is derived from CD11a.

In some embodiments, the sequence of the intracellular signal region is derived from CD18.

### BRIEF DESCRIPTION OF THE DRAWINGS

Fig. 1 is the performance curves of the binding of mHmL, H1L1, H2L1, H3L1, H3L2, H4L1, H5L1, H2L2, H2L3, H2L4, and H3L3 with SW480;
Fig. 2 is the performance curves of the binding of H1L4 and H1L3 with SW480;
Fig. 3 is the performance curves of the binding of mHmL, H1L1, H2L1, H3L1, H3L2, H4L1, H5L1, H2L2, H2L3, H2L4, H3L3 with HCT116;
Fig. 4 is the performance curves of the binding of H1L4 and H1L3 with HCT116;
Fig. 5 is the structural representation of the EpCAM-targeting chimeric antigen receptor;

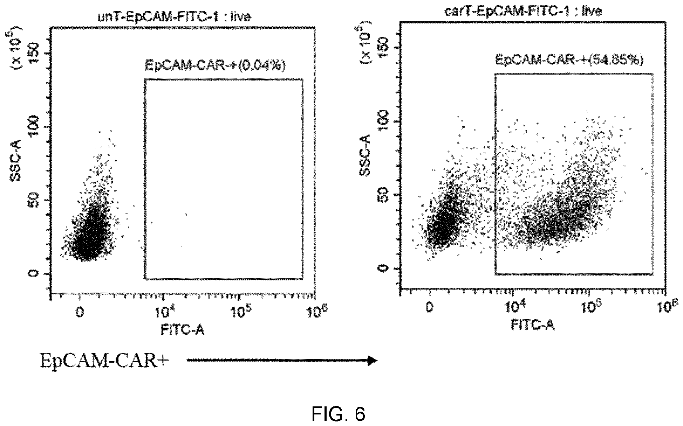

Fig. 6 is a comparison chart of the flow cytometry analysis results of the reference sample and CAR-T;
Fig. 7 is a comparison chart of the EpCAM expression levels of HCT116-Luc, SW480, KATO-III, A549 and MIAPaCA-2;
Fig. 8 is a comparison chart of the killing efficiency of CAR-T and reference T cell unT on different tumor cells at different effector/target ratios;
Fig. 9 is a comparison chart of the amount of IFN-γ released after the activation of CAR-T and reference T cells unT by different tumor cells;
Fig. 10 is a comparison chart of the change in average tumor volume of the mice in each group within the experiment;
Fig. 11 is a comparison chart of the change in average body weight of the mice in each group during the experiment;
Fig. 12 shows the results of histological anatomy of the mice in G1, G2 and G3 groups.

### DETAILED DESCRIPTION

In order to make the purposes, technical solutions and advantages of the embodiments of the present disclosure clearer, the technical solutions in the embodiments of the present disclosure will be described clearly and completely below. Obviously, the described embodiments are part of the embodiments of the present disclosure, but not all of them. Based on the embodiments of the present disclosure, all other embodiments obtained by those of ordinary skill in the art without creative efforts shall fall within the protection scope of the present disclosure. Unless otherwise defined, technical or scientific terms used herein should have the ordinary meaning as understood by one of ordinary skill in the art to which this disclosure belongs. As used herein, "comprising" and similar words mean that the elements or things appearing before the word encompass the elements or things recited after the word and their equivalents, but do not exclude other elements or things.

The technical and scientific terms used in the present disclosure have the same or similar meanings as commonly understood by those skilled in the art. To facilitate understanding of the present disclosure, some terms are defined as follows:

The "antibody" in the present disclosure refers to an antigen-binding protein of the immune system, including a complete full-length antibody with an antigen-binding region or any fragment of its "antigen-binding portion" or "antigen-binding region", or a single chain thereof such as a single chain variable fragment (scFv). A natural antibody refers to a glycoprotein of at least two heavy (H) chains and two light (L) chains or antigen-binding fragments thereof interconnected by disulfide bonds. The "antibody" also includes all recombinant forms of antibodies, particularly those described herein, such as antibodies expressed in prokaryotic cells, unglycosylated antibodies, and antibody fragments and derivatives that bind to an antigen. Each heavy chain consists of a heavy chain variable region (abbreviated as VH) and a heavy chain constant region (abbreviated as CH). Each light chain consists of a light chain variable region (abbreviated as VL) and a light chain constant region (abbreviated as CL). VH and VL can be further subdivided into hypervariable regions called complementarity determining regions (CDRs) interspersed in more conserved regions called framework regions (FRs). Each VH and VL consists of three CDRs and four FRs, arranged from amino-terminus to carboxy-terminus in the following order: FR1, CDR1, FR2, CDR2, FR3, CDR3, FR4. The variable regions of the heavy and light chains contain binding domains that interact with the antigen. The constant region of the antibody can mediate the binding of the immunoglobulin to host tissues or factors including various cells of the immune system (e.g., effector cells) and the first component (C1q) of the classical complement system.

Antibody fragments include, but are not limited to:
(i) a Fab fragment composed of VL, VH, CL and CH1 domains, including Fab' and Fab'-SH, (ii) an Fd fragment composed of VH and CH1 domains, (iii) an Fv fragment composed of VL and VH domains of a single antibody; (iv) a dAb fragment composed of a single variable region; (v) a F(ab')2 fragment, a bivalent fragment comprising 2 linked Fab fragments; (vi) a single-chain Fv molecule antigen binding site; (vii) a bispecific single-chain Fv dimer; (viii) a "disome" or a "trisome", a multivalent or multispecific fragment constructed by genetic fusion and (ix) an scFv genetically fused to the same or different antibodies.

The term "Fc" or "Fc region" in the present disclosure includes a polypeptide of the antibody constant region other than the immunoglobulin domain of the first constant region. Thus, Fc refers to the last two constant region immunoglobulin domains of IgA, IgD and IgG, and the last three constant region immunoglobulin domains of IgE and IgM, and the N-terminal flexible hinge of these domains. For IgA and IgM, Fc may include the J chain. For IgG, Fc includes the immunoglobulin domains Cγ2 and Cγ3 and a hinge between Cγ1 and Cγ2. Although the boundaries of the Fc region may vary, the Fc region of a human IgG heavy chain is generally defined as comprising residues C226 or P230 at its carboxy terminus, where the numbering is according to the Kabat EU index. For human IgG1, Fc is defined herein as comprising residue P232 to its carboxy terminus, where the numbering is according to the EU index in Kabat. Fc can refer to this region in isolation, or in the context of an Fc polypeptide, e.g., an antibody. The aforementioned "hinge" includes a flexible polypeptide comprising amino acids between the first and second constant domains of an antibody. Structurally, the CH1 domain of IgG ends at EU220 and the CH2 domain of IgG begins at residue EU237.

The term "mutated sequence obtained by substitution" in the present disclosure means replacing an amino acid at a specific position in the parent polypeptide sequence with another amino acid. The "deletion" in the "mutated sequence obtained by substitution or deletion" of the present disclosure means the removal of an amino acid at a specific position in the parent polypeptide sequence.

The "chimeric antigen receptor" or "CAR" of the present disclosure refers to those comprising an extracellular antigen binding region capable of binding an antigen, a hinge region, a transmembrane region and an intracellular signal region. The intracellular signal region refers to a protein that transmits information into a cell to regulate cell activity via an identified signal transduction pathway by producing a second messenger, or a protein that functions as an effector in response to such messengers, which comprises a primary signaling domain, and may also include a functional signaling domain (i.e., a costimulatory signaling domain) derived from a stimulatory molecule as defined below. Intracellular signaling domains generate signals that can promote the immunological effect subfunction of the cells of CAR (e.g., CAR-T cells), examples of the immunological effect subfunction, such as in CAR-T cells, include cytolytic activity and helper activity, including cellular factor secretion.

The term "costimulatory signaling domain" refers to a "costimulatory molecule", which is a cognate binding partner on a T cell that specifically binds a costimulatory ligand, thereby mediating a costimulatory response of the T cell, such as, but not limited to, proliferation. Costimulatory molecules are cell surface molecules or ligands thereof of non-antigen receptors, which are required for an effective immune response. Costimulatory molecules include, but are not limited to, Toll-like receptors, CD2, CD27, LFA-1 (CD11a/CD18), CD8α, CD28, 4-1BB, ICOS, OX40, CD40, CD80, DAP10, DAP12, CD3ζ, CD3ε, etc.

In the present disclosure:
"CD3ζ" is defined as the protein provided as GenBan Accession No. BAG36664.1, or the equivalent residues from a non-human species such as mouse, rodent, monkey, ape, and the like. A"CD3ζ domain" is defined as the amino acid residues from the cytoplasmic domain of the ζ chain sufficient to functionally transmit the initial signals required for T cell activation. In one aspect, the cytoplasmic domain of ζ comprises residues 52 to 163 of GenBank Accession No. BAG36664.1, functionally directed to homologues-equivalent residues from a non-human species such as mouse, rodent, monkey, ape, and the like.

"CD28" is defined as the protein provided as GenBan Accession No. NP_006130.1, or the equivalent residues from a non-human species such as mouse, rodent, monkey, ape, and the like. A "CD28 signaling region" is defined as the amino acid residues from the cytoplasmic domain of CD28, which is capable of transmitting costimulatory signals required for T cell activation, and the sequence of which comprises residues 180 to 220 of GenBank Accession No. NP_006130.1, functionally directed to homologues-equivalent residues from a non-human species such as mouse, rodent, monkey, ape, and the like. A "CD28 hinge region" comprises residues 114 to 152 of GenBank Accession No. NP_006130.1, functionally directed to homologues-equivalent residues from a non-human species such as mouse, rodent, monkey, ape, and the like. A "CD28 hinge transmembrane region" comprises residues 153 to 179 of GenBank Accession No. NP_006130.1, functionally directed to homologues-equivalent residues from a non-human species such as mouse, rodent, monkey, ape, and the like.

"4-1BB" refers to a member of the TNFR superfamily having the amino acid sequence of GenBank Accession No. NP_001552.2, or equivalent residues from a non-human species such as mouse, rodent, monkey, ape, and the like. A "4-1BB signaling region" is defined as the amino acid residues from the cytoplasmic domain of 4-1BB, which is capable of transmitting costimulatory signals required for T cell activation, and the sequence of which comprises residues 214 to 255 of GenBank Accession No. NP_006130.1 255, functionally directed to homologues-equivalent residues from a non-human species such as mouse, rodent, monkey, ape, and the like.

EpCAM described in the embodiments of the present disclosure is an epithelial cell adhesion molecule. The humanized antibody specifically binds to EpCAM expressed in relevant tumors. The relevant tumors include epithelial tumors, specifically esophageal cancer, gastric cancer, colorectal cancer, prostate cancer, lung cancer, ovarian cancer, breast cancer, kidney cancer, hepatocellular carcinoma and pancreatic cancer.

In an embodiment of the present disclosure, the humanized antibody is a humanized full-length antibody, which has a subtype of hIgG1.

In some embodiments, the humanized antibody is a single-chain antibody, which has a subtype of any one of hIgG1, hIgG2, hIgG3 and hIgG4.

In some embodiments, the heavy chain variable region sequence includes a sequence shown in SEQ ID NO: 1, a sequence shown in SEQ ID NO: 2, and a sequence shown in SEQ ID NO: 3, respectively denoted as a sequence of CDR-H1, a sequence of CDR-H2 and a sequence of CDR-H3, CDR-H1, CDR-H2 and CDR-H3 are a first heavy chain complementarity determining region, a second heavy chain complementarity determining region and a third heavy chain complementarity determining region, respectively.

In some embodiments, a sequence of a first heavy chain framework region between CDR-H1 and CDR-H2 is QVQLVQSGAEVKKPG X6SVKVSCKASGX7TFX8, where X6 is any one of A and S, X7 is any one of Y and G, and X8 is any one of T and S.

In some embodiments, a sequence of a second heavy chain framework region between CDR-H2 and CDR-H3 is any one of WVRQAPGQGLEWMG and WVRQAPGQGLEWIG.

In some embodiments, a sequence of a third heavy chain framework region connecting CDR-H3 is X9VTX10TX11DX12STSTX13YMELSSLRSEDTAVYYCAR, X9 is any one of R and K, X10 is any one of M, L and I, X11 is any one of R, T, A and V, X12 is any one of T and E, and X13 is any one of V and A.

In some embodiments, the light chain variable region sequence includes a sequence shown in SEQ ID NO: 4, a sequence shown in SEQ ID NO: 5 and a sequence shown in SEQ ID NO: 6, respectively denoted as a sequence of CDR-L1, a sequence of CDR-L2 and a sequence of CDR-L3. CDR-L1, CDR-L2 and CDR-L3 are a first light chain complementarity determining region, a second light chain complementarity determining region and a third light chain complementarity determining region, repectively.

An embodiment of the present disclosure also provides a chimeric antigen receptor specifically targeting EpCAM, the antigen binding region of the chimeric antigen receptor comprises the humanized antibody, or a fragment of the humanized antibody.

Preferably, in some embodiments, the EpCAM-targeting antibody fragments a single-chain variable region (scFv); the single-chain variable region has a structure of VH-Linker-VL or VL-Linker-VH; the amino acid sequence of the Linker is shown in SEQ ID NO.30, which is GGGGSGGGGSGGGGS;

In some embodiments, the chimeric antigen receptor further comprises a hinge region, the sequence of which is derived from at least one of CD8α, CD28, 4-1BB, ICOS, OX40, CD40, CD80 and IgG.

In some embodiments, the chimeric antigen receptor further comprises a transmembrane region, the sequence of which is derived from at least one of CD8α, CD28, 4-1BB, ICOS, OX40, CD40, CD80 and CD3.

In some embodiments, the chimeric antigen receptor further comprises an intracellular signal region, the sequence of which is derived from at least one of CD8α, CD28, 4-1BB, ICOS, OX40, CD40, CD80, DAP10, DAP12, CD3ζ and CD3ε.

Preferably, the CAR molecular sequence is shown in the following table:

**Table 1 Sequences of CAR Molecules Specifically Targeting EpCAM**

| SEQ ID | scFv | CAR sequences |
|---|---|---|
| 18 | H1L1 | MLLLVTSLLLCELPHPAFLLIPQVQLVQSGAEVKKPGASVKLSCKASGYTFT NYWINWVKQAPGQGLEWIGNIYPSYIYTNYNQEFKDKVTLTVDESTSTAYMEL SSLRSEDTAVYYCTRSPYGYDEYGLDYWGQGTTVTVSSGGGGSGGGGSGG GGSDIQLTQSPSSLSASVGDRVTMTCKSSQSLLNSRNQKNYLTWYQQKPGKA PKLLIYWASTRESGVPSRFSGSGSGTDFTLTISSVQPEDFATYYCQNDYVYPL TFGQGTKLEIKIEVMYPPPYLDNEKSNGTIIHVKGKHLCPSPLFPGPSKPFWVL VVVGGVLACYSLLVTVAFIIFWVRSKRSRLLHSDYMNMTPRRPGPTRKHYQPY APPRDFAAYRSRVKFSRSADAPAYQQGQNQLYNELNLGRREEYDVLDKRRG RDPEMGGKPRRKNPQEGLYNELQKDKMAEAYSEIGMKGERRRGKGHDGLY QGLSTATKDTYDALHMQALPPR |
| 19 | H2L1 | MLLLVTSLLLCELPHPAFLLIPQVQLVQSGAEVKKPGASVKVSCKASGYTFT NYWINWVRQAPGQGLEWIGNIYPSYIYTNYNQEFKDKVTLTVDESTSTAYMEL SSLRSEDTAVYYCTRSPYGYDEYGLDYWGQGTTVTVSSGGGGSGGGGSGG GGSDI QLTQSPSSLSASVGDRVTMTCKSSQSLLNSRNQKNYLTWYQQKPGKAPKLLI YWASTRESGVPSRFSGSGSGTDFTLTISSVQPEDFATYYCQNDYVYPLTFGQ GTKLEIKIEVMYPPPYLDNEKSNGTIIHVKGKHLCPSPLFPGPSKPFWVLVVVG GVLACYSLLVTVAFIIFWVRSKRSRLLHSDYMNMTPRRPGPTRKHYQPYAPPR DFAAYRSRVKFSRSADAPAYQQGQNQLYNELNLGRREEYDVLDKRRGRDPE MGGKPRRKNPQEGLYNELQKDKMAEAYSEIGMKGERRRGKGHDGLYQGLST ATKDTYDALHMQALPPR |
| 20 | H3L1 | MLLLVTSLLLCELPHPAFLLIPQVQLVQSGAEVKKPGASVKVSCKASGYTFT NYWINWVRQAPGQGLEWIGNIYPSYIYTNYNQEFKDRVTLTRDTSISTAYMEL SRLRSDDTAVYYCTRSPYGYDEYGLDYWGQGTTVTVSSGGGGSGGGGSGG GGSDIQLTQSPSSLSASVGDRVTMTCKSSQSLLNSRNQKNYLTWYQQKPGKA PKLLIYWASTRESGVPSRFSGSGSGTDFTLTISSVQPEDFATYYCQNDYVYPL TFGQGTKLEIKIEVMYPPPYLDNEKSNGTIIHVKGKHLCPSPLFPGPSKPFWVL VVVGGVLACYSLLVTVAFIIFWVRSKRSRLLHSDYMNMTPRRPGPTRKHYQPY APPRDFAAYRSRVKFSRSADAPAYQQGQNQLYNELNLGRREEYDVLDKRRG RDPEMGGKPRRKNPQEGLYNELQKDKMAEAYSEIGMKGERRRGKGHDGLY QGLSTATKDTYDALHMQALPPR |
| 21 | H3L2 | MLLLVTSLLLCELPHPAFLLIPQVQLVQSGAEVKKPGASVKVSCKASGYTFT NYWINWVRQAPGQGLEWIGNIYPSYIYTNYNQEFKDRVTLTRDTSISTAYMEL SRLRSDDTAVYYCTRSPYGYDEYGLDYWGQGTTVTVSSGGGGSGGGGSGG GGSDIQLTQSPSSLSASVGDRVTMTCKSSQSLLNTRNQKNYLTWYQQKPGKA PKLLIYWASTRESGVPSRFSGSGSGTDFTLTISSLQPEDFATYYCQNDYVYPLT FGQGTKLEIKIEVMYPPPYLDNEKSNGTIIHVKGKHLCPSPLFPGPSKPFWVLV VVGGVLACYSLLVTVAFIIFWVRSKRSRLLHSDYMNMTPRRPGPTRKHYQPYA PPRDFAAYRSRVKFSRSADAPAYQQGQNQLYNELNLGRREEYDVLDKRRGR DPEMGGKPRRKNPQEGLYNELQKDKMAEAYSEIGMKGERRRGKGHDGLYQ |
| | | GLSTATKDTYDALHMQALPPR |
| 22 | H4L1 | MLLLVTSLLLCELPHPAFLLIPQVQLVQSGAEVKKPGASVKVSCKASGYTFT NYWINWVRQAPGQGLEWIGNIYPSYIYTNYAQKFQGRVTLTRDTSTSTVYMEL SSLRSEDTAVYYCTRSPYGYDEYGLDYWGQGTTVTVSSGGGGSGGGGSGG GGSDIQLTQSPSSLSASVGDRVTMTCKSSQSLLNSRNQKNYLTWYQQKPGKA PKLLIYWASTRESGVPSRFSGSGSGTDFTLTISSVQPEDFATYYCQNDYVYPL TFGQGTKLEIKIEVMYPPPYLDNEKSNGTIIHVKGKHLCPSPLFPGPSKPFWVL VVVGGVLACYSLLVTVAFIIFWVRSKRSRLLHSDYMNMTPRRPGPTRKHYQPY APPRDFAAYRSRVKFSRSADAPAYQQGQNQLYNELNLGRREEYDVLDKRRG RDPEMGGKPRRKNPQEGLYNELQKDKMAEAYSEIGMKGERRRGKGHDGLY QGLSTATKDTYDALHMQALPPR |
| 23 | H5L1 | MLLLVTSLLLCELPHPAFLLIPQVQLVQSGAEVKKPGASVKVSCKASGYTFT NYWINWVRQAPGQGLEWIGNIYPSYIYTNYNQEFKDRVTMTRDTSISTAYMEL SRLRSDDTAVYYCTRSPYGYDEYGLDYWGQGTTVTVSSGGGGSGGGGSGG GGSDIQLTQSPSSLSASVGDRVTMTCKSSQSLLNSRNQKNYLTWYQQKPGKA PKLLIYWASTRESGVPSRFSGSGSGTDFTLTISSVQPEDFATYYCQNDYVYPL TFGQGTKLEIKIEVMYPPPYLDNEKSNGTIIHVKGKHLCPSPLFPGPSKPFWVL VVVGGVLACYSLLVTVAFIIFWVRSKRSRLLHSDYMNMTPRRPGPTRKHYQPY APPRDFAAYRSRVKFSRSADAPAYQQGQNQLYNELNLGRREEYDVLDKRRG RDPEMGGKPRRKNPQEGLYNELQKDKMAEAYSEIGMKGERRRGKGHDGLY QGLSTATKDTYDALHMQALPPR |
| 24 | H2L2 | MLLLVTSLLLCELPHPAFLLIPQVQLVQSGAEVKKPGASVKVSCKASGYTFTNY WINWVRQAPGQGLEWIGNIYPSYIYTNYNQEFKDKVTLTVDESTSTAYMELSS LRSEDTAVYYCTRSPYGYDEYGLDYWGQGTTVTVSSGGGGSGGGGSGGGG SDIQLTQSPSSLSASVGDRVTMTCKSSQSLLNTRNQKNYLTWYQQKPGKAPK LLIYWASTRESGVPSRFSGSGSGTDFTLTISSLQPEDFATYYCQNDYVYPLTF GQGTKLEIKIEVMYPPPYLDNEKSNGTIIHVKGKHLCPSPLFPGPSKPFWVLVV VGGVLACYSLLVTVAFIIFWVRSKRSRLLHSDYMNMTPRRPGPTRKHYQPYAP PRDFAAYRSRVK FSRSADAPAYQQGQNQLYNELNLGRREEYDVLDKRRGRDPEMGGKPRRKN |
| | | PQEGLYNELQKDKMAEAYSEIGMKGERRRGKGHDGLYQGLSTATKDTYDAL HMQALPPR |
| 25 | H2L3 | MLLLVTSLLLCELPHPAFLLIPQVQLVQSGAEVKKPGASVKVSCKASGYTFT NYWINWVRQAPGQGLEWIGNIYPSYIYTNYNQEFKDKVTLTVDESTSTAYMEL SSLRSEDTAVYYCTRSPYGYDEYGLDYWGQGTTVTVSSGGGGSGGGGSGG GGSDIVLTQSPDSLAVSLGERATMNCKSSQSLLNSRNQKNYLTWYQQKPGQP PKLLIYWASTRESGVPDRFSGSGSGTDFTLTISSLQAEDVAVYYCQNDYVYPL TFGQGTKLEIKIEVMYPPPYLDNEKSNGTIIHVKGKHLCPSPLFPGPSKPFWVL VVVGGVLACYSLLVTVAFIIFWVRSKRSRLLHSDYMNMTPRRPGPTRKHYQPY APPRDFAAYRSRVKFSRSADAPAYQQGQNQLYNELNLGRREEYDVLDKRRG RDPEMGGKPRRKNPQEGLYNELQKDKMAEAYSEIGMKGERRRGKGHDGLY QGLSTATKDTYDALHMQALPPR |
| 26 | H2L4 | MLLLVTSLLLCELPHPAFLLIPQVQLVQSGAEVKKPGASVKVSCKASGYTFT NYWINWVRQAPGQGLEWIGNIYPSYIYTNYNQEFKDKVTLTVDESTSTAYMEL SSLRSEDTAVYYCTRSPYGYDEYGLDYWGQGTTVTVSSGGGGSGGGGSGG GGSEIVLTQSPATLSLSPGERATMSCKSSQSLLNSRNQKNYLTWYQQKPGQA PRLLIYWASTRESGIPARFSGSGSGTDFTLTISSLEPEDFAVYYCQNDYVYPLT FGQGTKLEIKIEVMYPPPYLDNEKSNGTIIHVKGKHLCPSPLFPGPSKPFWVLV VVGGVLACYSLLVTVAFIIFWVRSKRSRLLHSDYMNMTPRRPGPTRKHYQPYA PPRDFAAYRSRVKFSRSADAPAYQQGQNQLYNELNLGRREEYDVLDKRRGR DPEMGGKPRRKNPQEGLYNELQKDKMAEAYSEIGMKGERRRGKGHDGLYQ GLSTATKDTYDALHMQALPPR |
| 27 | H3L3 | MLLLVTSLLLCELPHPAFLLIPQVQLVQSGAEVKKPGASVKVSCKASGYTFT NYWINWVRQAPGQGLEWIGNIYPSYIYTNYNQEFKDRVTLTRDTSISTAYMEL SRLRSDDTAVYYCTRSPYGYDEYGLDYWGQGTTVTVSSGGGGSGGGGSGG GGSDIVLTQSPDSLAVSLGERATMNCKSSQSLLNSRNQKNYLTWYQQKPGQP PKLLIY WASTRESGVPDRFSGSGSGTDFTLTISSLQAEDVAVYYCQNDYVYPLTFGQG TKLEIKIEVMYPPPYLDNEKSNGTIIHVKGKHLCPSPLFPGPSKPFWVLVVVGG VLACYSLLVTVAFIIFWVRSKRSRLLHSDYMNMTPRRPGPTRKHYQPYAPPRD |
| | | FAAYRSRVKFSRSADAPAYQQGQNQLYNELNLGRREEYDVLDKRRGRDPEM GGKPRRKNPQEGLYNELQKDKMAEAYSEIGMKGERRRGKGHDGLYQGLSTA TKDTYDALHMQALPPR |
| 28 | H1L4 | MLLLVTSLLLCELPHPAFLLIPQVQLVQSGAEVKKPGASVKLSCKASGYTFT NYWINWVKQAPGQGLEWIGNIYPSYIYTNYNQEFKDKVTLTVDESTSTAYMEL SSLRSEDTAVYYCTRSPYGYDEYGLDYWGQGTTVTVSSGGGGSGGGGSGG GGSEIVLTQSPATLSLSPGERATMSCKSSQSLLNSRNQKNYLTWYQQKPGQA PRLLIYWASTRESGIPARFSGSGSGTDFTLTISSLEPEDFAVYYCQNDYVYPLT FGQGTKLEIKIEVMYPPPYLDNEKSNGTIIHVKGKHLCPSPLFPGPSKPFWVLV VVGGVLACYSLLVTVAFIIFWVRSKRSRLLHSDYMNMTPRRPGPTRKHYQPYA PPRDFAAYRSRVKFSRSADAPAYQQGQNQLYNELNLGRREEYDVLDKRRGR DPEMGGKPRRKNPQEGLYNELQKDKMAEAYSEIGMKGERRRGKGHDGLYQ GLSTATKDTYDALHMQALPPR |
| 29 | H1L3 | MLLLVTSLLLCELPHPAFLLIPQVQLVQSGAEVKKPGASVKLSCKASGYTFT NYWINWVKQAPGQGLEWIGNIYPSYIYTNYNQEFKDKVTLTVDESTSTAYMEL SSLRSEDTAVYYCTRSPYGYDEYGLDYWGQGTTVTVSSGGGGSGGGGSGG GGSDIVLTQSPDSLAVSLGERATMNCKSSQSLLNSRNQKNYLTWYQQKPGQP PKLLIYWASTRESGVPDRFSGSGSGTDFTLTISSLQAEDVAVYYCQNDYVYPL TFGQGTKLEIKIEVMYPPPYLDNEKSNGTIIHVKGKHLCPSPLFPGPSKPFWVL VVVGGVLACYSLLVTVAFIIFWVRSKRSRLLHSDYMNMTPRRPGPTRKHYQPY APPRDFAAYRSRVKFSRSADAPAYQQGQNQLYNELNLGRREEYDVLDKRRG RDPEMGGKPRRKNPQEGLYNELQKDKMAEAYSEIGMKGERRRGKGHDGLY QGLSTATKDTYDALHMQALPPR |

An embodiment of the present disclosure also provide a use of the chimeric antigen receptor, including specifically targeting the EpCAM antigen that is highly expressed in relevant tumors.

An embodiment of the present disclosure also provides a nucleic acid comprising a nucleotide sequence encoding the chimeric antigen receptor.

An embodiment of the present disclosure also provides a vector comprising the nucleic acid.

An embodiment of the present disclosure also provides a cell comprising the vector.

### Example 1. Design of Humanized Antibody

The example provides a method for designing the humanized antibody, including the following steps:
S1: a murine antibody VH sequence shown in SEQ ID NO.16 and a murine antibody VL sequence shown in SEQ ID NO.17 were obtained;
S2: the murine antibody sequences were compared with human antibody database to find a human antibody germline with the highest homology;
S3: computer modeling was performed by simulating the sites in the antibody structure that may affect the binding with antigens, key back-mutation sites and combinations, and then screening out the optimal structural solution.

Where, IGHV1 with the highest homology was selected as the humanized design template for the design of VH, and IGKV1, IGKV3 and IGKV4 were preferentially selected as the humanized design templates for the design of VL. A common pairing method was used for IGHV1-KV1 and IGHV1-KV3.

Where, 5-10 optimal structural solutions were selected by Discovery Studio and Schrödinger Antibody Modeling in a homology modeling method.

Specifically, the Loop region was generally modeled by the homology modeling method. If the CDR amino acid sequence alignment result showed that the homology was lower than 50%, a de novo modeling method was used to build the CDR3 structure model. PDB BLAST was used to retrieve 10 antibody crystal structure models with a structural resolution higher than 2.5 angstroms that are closest to the sequence, and the optimal structural model was selected by comparing with the automatic modeling models.

The sequences of the above-mentioned humanized antibodies were aligned with the human Germline sequences, and the obtained percentage of humanization degree and the number of back-mutation sites are shown in Table 2.

**Table 2 Humanization Degree Score of Each Humanized Antibody**

| Humanized Antibody Name | Humanization Degree % | Number of Back-mutation Sites |
|---|---|---|
| H1L1 | 98.5% | 10 |
| H2L1 | 98.8% | 8 |
| H3L1 | 99.1% | 6 |
| H3L2 | 99.3% | 5 |
| H4L1 | 99.15 | 6 |
| H5L1 | 99.3% | 5 |
| H2L2 | 99.0% | 7 |
| H2L3 | 99.0% | 7 |
| H2L4 | 99.1% | 6 |
| H3L3 | 99.3% | 5 |
| H1L4 | 98.8% | 8 |
| H1L3 | 98.7% | 9 |

### Example 2. Expression and Purification of Humanized Antibody and Detection of EpCAM Specific Binding Ability

In the example of the present disclosure, the use of the humanized antibody includes using pCDNA3.4 vector and ExpiCHO-s expression system to express the humanized antibody.

Further, each sequence of SEQ ID NO: 7-11 was cloned into pCDNA3.4 vector to obtain several heavy chain gene expression vectors, and each sequence of SEQ ID NO: 12-15 was cloned into pCDNA3.4 vector to obtain several light chain gene expression vectors, and then the heavy chain gene expression vectors and the light chain gene expression vectors were paired and respectively transfected into the ExpiCHO-s expression system to obtain several recombinant antibodies, which were denoted as H1L1, H2L1, H3L1, H3L2, H4L1, H5L1, H2L2, H2L3, H2L4, H3L3, H1L4, and H1L3. mHmL is the sequence of the EpCAM antibody. The specific cloning and transfection processes are conventional technical means of those skilled in the art and will not be repeated here.

Further, the present disclosure determines the binding and dissociation curves of empty probe, mHmL, H1L1, H2L1, H3L1, H3L2, H4L1, H5L1, H2L2, H2L3, H2L4, H3L3, H1L4 and H1L3 with Human-EpCAM-Protetin-His, respectively. The specific determination method was: a PBST buffer with a concentration of 0.025% formed by dissolving Tween 20 in PBS buffer was used to dilute each recombinant antibody to a concentration of 50 nM, and Human-EpCAM-Protetin-His to a concentration gradient from 500 nM to 31.3 nM. Protein A was coupled to a biosensor and activated using the above PBST buffer for 10 min; the loading time was 120 s, the binding time was 300 s, and the dissociation time was 300 s. The results are shown in Table 3 as the affinity constant KD value. As can be seen from Table 3, the affinity constant of the recombinant antibody to the EpCAM antigen was 1.0×10⁻⁸ mol-10⁻¹⁰ mol.

**Table 3 Affinity of Each antibody to EpCAM**

| Recombinant Antibody Name | KD (mole) |
|---|---|
| mHmL | 1.67E-08 |
| H1L1 | 1.87E-08 |
| H2L1 | 1.88E-08 |
| H3L1 | 2.19E-08 |
| H3L2 | 6.47E-09 |
| H4L1 | 8.19E-09 |
| H5L1 | 1.77E-08 |
| H2L2 | 4.66E-09 |
| H2L3 | 1.14E-08 |
| H2L4 | 1.34E-08 |
| H3L3 | 1.41E-08 |
| H1L4 | 1.18E-08 |
| H1L3 | 9.74E-09 |

In the examples of the present disclosure, the binding activities of the recombinant antibodies to human colon cancer cells SW480, human colorectal cancer cells HCT116 and colon cancer RKO cells were also investigated by flow cytometry fluorescence sorting. The results showed that each recombinant antibody did not bind well to RKO cells, but had good binding activity to human colon cancer cell SW460 and human colorectal cancer cell HCT116.

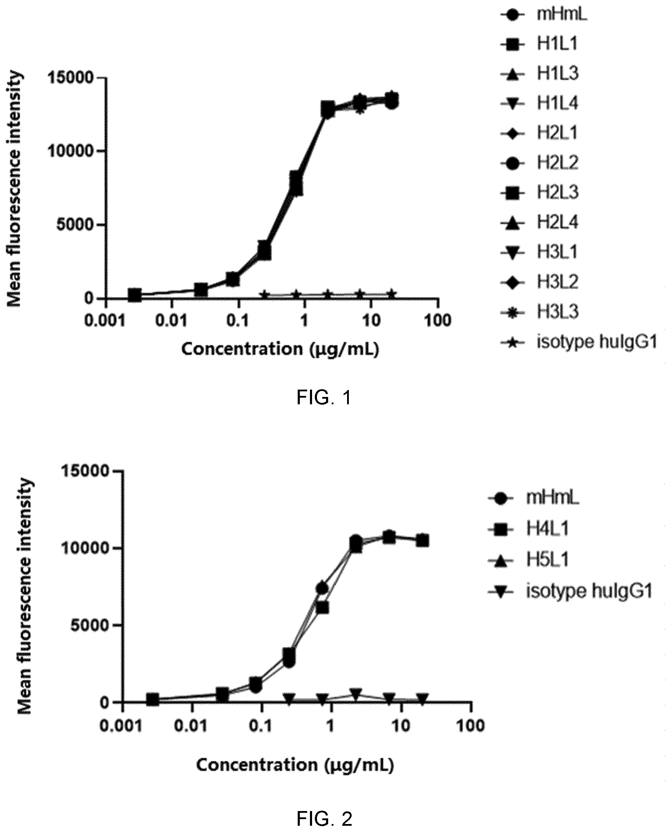

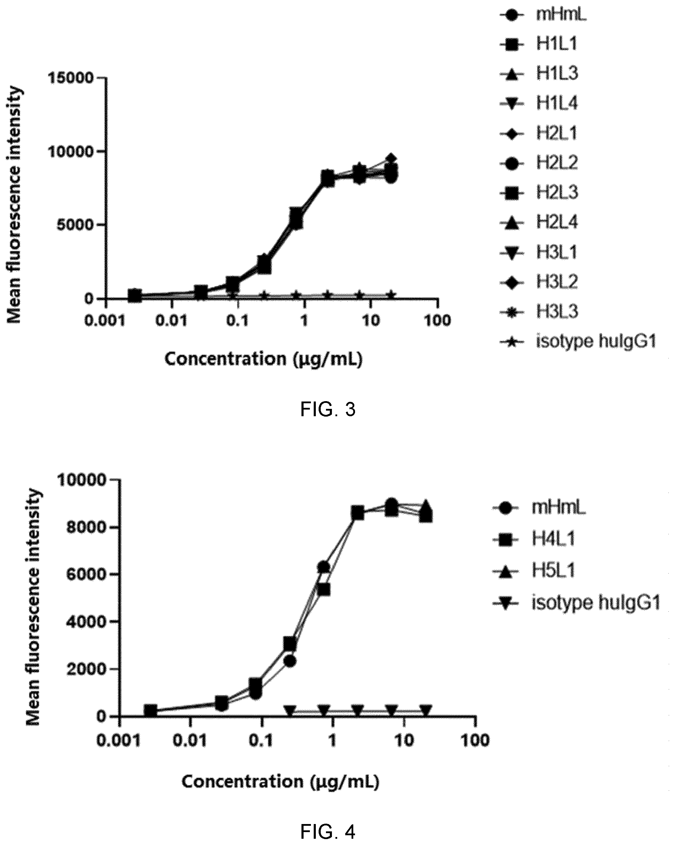

Specifically, the number of cells in each flow tube was 1×10⁵, and the cells were centrifuged and washed with FACS buffer; after resuspension, 100 µL of FACS diluent was added to each flow tube, and the cells were incubated at 4°C for 60 minutes; the cells were centrifuged and washed with FACS buffer 5 times, then 100µL of fluorescein FITC and Fcγ receptor fragments were added to each flow tube and the mixture was incubated at 4°C for 30 minutes; and finally, after being centrifugated and washed with FACS diluent, the cells were resuspended with FACS diluent for testing, and the performance curves of the binding of mHmL, H1L1, H2L1, H3L1, H3L2, H4L1, H5L1, H2L2, H2L3, H2L4, and H3L3 with SW480 as shown in Figure 1, and the performance curves of the binding of H1L4 and H1L3 with SW480 as shown in Figure 2, the performance curves of the binding of mHmL, H1L1, H2L1, H3L1, H3L2, H4L1, H5L1, H2L2, H2L3, H2L4, and H3L3 with HCT116 as shown in Figure 3, and the performance curves of the binding of H1L4 and H1L3 with HCT116 as shown in Figure 4, are obtained. The EC50 values of each recombinant antibody on SW480 and HCT116 are shown in Table 3, which are derived from Figures 1-4. As shown in Table 4, the EC50 of each humanized antibody binding to EpCAM was similar to that of the original antibody mHmL, indicating that the humanization modification did not significantly affect the affinity of the antibody.

**Table 4 Detection of the Ability of Each Antibody to Bind to Target Cells by Flow Cytometry**

| Recombinant Antibody Name | EC50 on SW480 (µg / ml) | EC50 on HCT116 (µg / ml) |
|---|---|---|
| mHmL | 0.5809 | 0.4677 |
| H1L1 | 0.5667 | 0.5032 |
| H2L1 | 0.5989 | 0.583 |
| H3L1 | 0.5642 | 0.4778 |
| H3L2 | 0.5685 | 0.4725 |
| H4L1 | 0.5675 | 0.4743 |
| H5L1 | 0.4491 | 0.4259 |
| H2L2 | 0.5951 | 0.537 |
| H2L3 | 0.6526 | 0.5837 |
| H2L4 | 0.6546 | 0.5839 |
| H3L3 | 0.5419 | 0.4924 |
| H1L4 | 0.6754 | 0.6021 |
| H1L3 | 0.6311 | 0.5634 |

### Example 3. Molecular Design and Lentiviral Packaging of EpCAM-targeting CAR

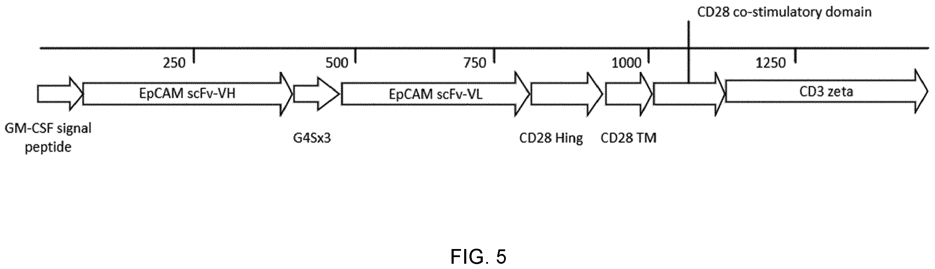

The example of the present disclosure provides a EpCAM-targeting chimeric antigen receptor (abbreviated as EpCAM-CAR), the structure of which is shown in FIG. 5, comprising a signal peptide, an antigen binding region, a hinge region, a transmembrane region and a intracellular costimulatory signaling domain.

Specifically, the sequence of EpCAM-CAR is shown in SEQ ID NO: 19, see SEQ ID NO:19:

The sequence of GM-CSF signal peptide is MLLLVTSLLLCELPHPAFLLIP;

The VH sequence of the humanized antibody is:

QVQLVQSGAEVKKPGASVKVSCKASGYTFTNYWINWVRQAPGQGLEWIGNI
YPSYIYTNYNQEFKDKVTLTVDESTSTAYMELSSLRSEDTAVYYCTRSPYGYDEYGLD
YWGQGTTVTVSS;

The sequence of the G4S x3 linker region is: GGGGSGGGGSGGGGS;

The VL sequence of the humanized antibody is:

DIQLTQSPSSLSASVGDRVTMTCKSSQSLLNTRNQKNYLTWYQQKPGKAPK
LLIYWASTRESGVPSRFSGSGSGTDFTLTISSLQPEDFATYYCQNDYVYPLTFGQGTK
LEIK;

The sequence of CD28 hinge region is:
IEVMYPPPYLDNEKSNGTIIHVKGKHLCPSPLFPGPSKP;

The sequence of CD28 transmembrane region is: FWVLWVGGVLACYSLLVTVAFIIFW;

The sequence of CD28 costimulatory signaling region is:
RSKRSRLLHSDYMNMTPRRPGPTRKHYQPYAPPRDFAAYRS;

The sequence of CD3ζ costimulatory signaling region is:

RVKFSRSADAPAYQQGQNQLYNELNLGRREEYDVLDKRRGRDPEMGGKPR
RKNPQEGLYNELQKDKMAEAYSEIGMKGERRRGKGHDGLYQGLSTATKDTYDALHM
QALPPR.

In the example of the present disclosure, the lentiviral vector plasmid encoding EpCAM-CAR was packaged into lentivirus, and the CAR plasmid composed of EpCAM-CAR and the backbone part of the third-generation lentiviral vector comes from Heyuan Biotechnology Co., Ltd.

The specific packaging method was as follows:
293T cells were inoculated in a T75 culture flask and cultured until the confluency was about 70%-80%, then the medium was replaced with an equal volume of medium to obtain a sample to be transfected, where the number of the cells in the culture flask was 5× 10⁶, and the culture volume was 20 ml;
Tube A solution was prepared using 2 ml Opti-MEM and 55 µL Lipo3000; and Tube B solution was prepared using 2 ml Opti-MEM, 46 µL P3000, 18 µg auxiliary plasmid and 6 µg EpCAM-CAR main plasmid;
The Tube A and Tube B solutions were mixed, incubated at room temperature for 15 minutes, and then added to the sample to be transfected, and the mixture was incubated for 48 hours;
After 48 hours of transfection, the supernatant was collected and centrifuged at 500g for 10min. The supernatant was filtered and sealed in a centrifuge tube, centrifuged at 10,000g at 4°C overnight to obtain a white virus precipitate; and the white virus precipitate was extracted and dissolved in 200 µl AIM-V medium, and then 2µl was taken to measure the titer according to the subsequent steps, and the rest was stored at-80°C.

2µl of the resuspended virus supernatant was added to 198ul of 1640 medium to dilute the virus, and then 2 µl, 10 µl and 50 µl of the diluted virus were each added to one of 3 wells in a 24-well plate, and Polybrene helper virus was added at a final concentration of 5 µl/ml for infection for 48 hours to obtain the Lentivirus. After the virus infection, the virus titer was detected with EpCAM-FITC labeled antigens, and the titer was between 2.5E+07-1.2E+08.

### Example 4. Construction of EpCAM-targeting CAR-T cells

In the example of the present disclosure, the above lentivirus was used to infect human peripheral blood mononuclear cells (PBMC) to construct CAR-T cells (abbreviated as CAR-T). The specific construction process was as follows:

Human peripheral blood mononuclear cells (PBMC) were cultured in a medium consisting of AIM-V medium, 5% fetal bovine serum, 100 U/mL penicillin, 0.1 mg/mL streptomycin and 300 IU/mL IL-2; the CD2/CD3/CD28 T cell activation and expansion kit from Miltenyi Company was used to activate T cells, that is, coated magnetic beads were mixed with cells in a ratio of 1:2, and the final cell density was 5×10⁶ cells/mL/cm². After mixing, the mixture was placed in a 37°C, 5 % CO₂ incubator for stimulation for 48 hours; RetroNectin from Takara was diluted to 20 µg/ml and then coated on a culture plate (non-tissue culture treated), with a coating solution of 4 µg /cm², placed in a 4 °C refrigerator overnight. After 48h of T cell activation, the cells were centrifuged at 300 g for 5 min to remove the supernatant, the T cells were resuspended in fresh medium, transferred to the plate coated with RetroNectin from Takara, the above lentivirus was added at an MOI = 5, the plate was placed in a 37 °C, 5 % CO₂ incubator for culture; at the same time, T cells without lentivirus transfection were prepared as a reference sample, abbreviated as unT; and 24 hours after the lentivirus was added, the cells were centrifuged at 300 g for 5 min to remove the supernatant, and T cells were suspended with culture medium to obtain CAR-T.

Further, 48 hours after adding the lentivirus, the samples were taken and the transduction rate was detected by flow cytometry, in which EpCAM protein was used as the primary antibody, and anti-EpCAM-FITC from Biolegend Company was used as the secondary antibody. The comparison chart of the flow analysis results of untransduced T cells (unT) and CAR-T shows that CAR was successfully expressed on CAR-T.

### Example 5. Verification of the Ability of EpCAM-targeting CAR-T Cells to Kill Target Cells In Vitro

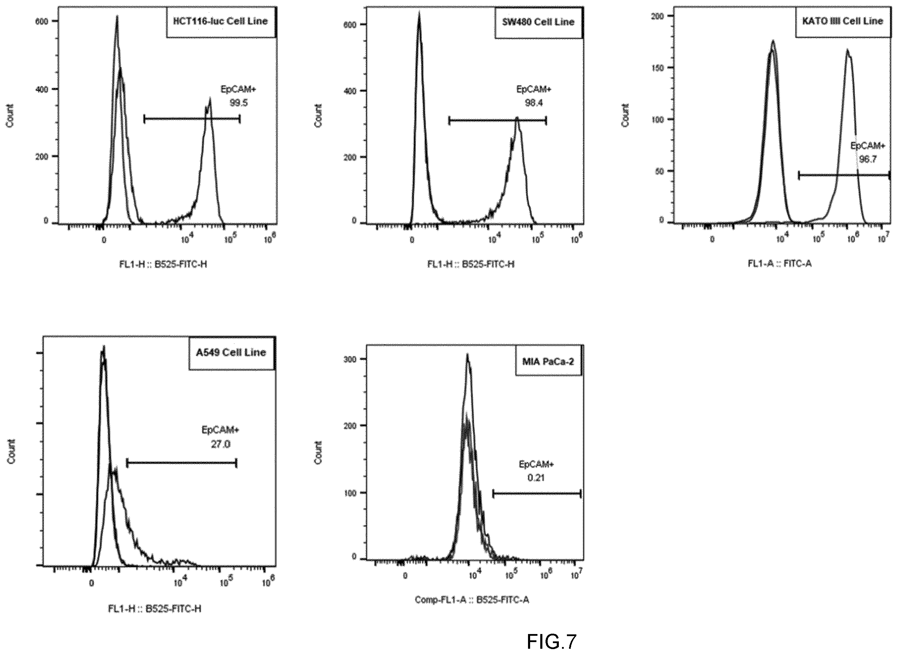

In the example of the present disclosure, tumor cell lines with different EpCAM expression conditions were selected to investigate the in vitro killing performance of CAR-T. Fig. 7 is a comparison chart of the EpCAM expression levels of HCT116-Luc, SW480, KATO-III, A549 and MIAPaCA-2. Referring to Fig. 7, it can be seen that the tumor cell lines with different EpCAM expression levels are respectively as below: the cell lines positively expressing EpCAM are HCT116-Luc, SW480, KATO-III and A549; and the cell line negatively expressing EpCAM is MIAPaCA-2.

The example of the present disclosure examined the in vitro killing effect of CAR-T on HCT116-Luc, SW480, KATO-III, A549 and MIAPaCA-2.

Specifically, in a 96-well plate, the prepared CAR-T or reference T cells (unT) were mixed with 1 × 10⁴ tumor cells according to different effector/target ratios, with 3 replicate wells set for each group, and placed in a 37 °C,5 % CO₂ incubator for cocultivation; 24 hours later, for HCT116-luc, SW480, KATO-III, A549, and MIAPaCA-2, the release of LDH from the target cells was detected using the LDH kit from Sigma, thereby obtaining the killing efficiency; and the supernatant of the co-culture well corresponding to the highest effector/target ratio was taken, and the amount of IFN-γ was detected by the HTRF kit from cisbio company.

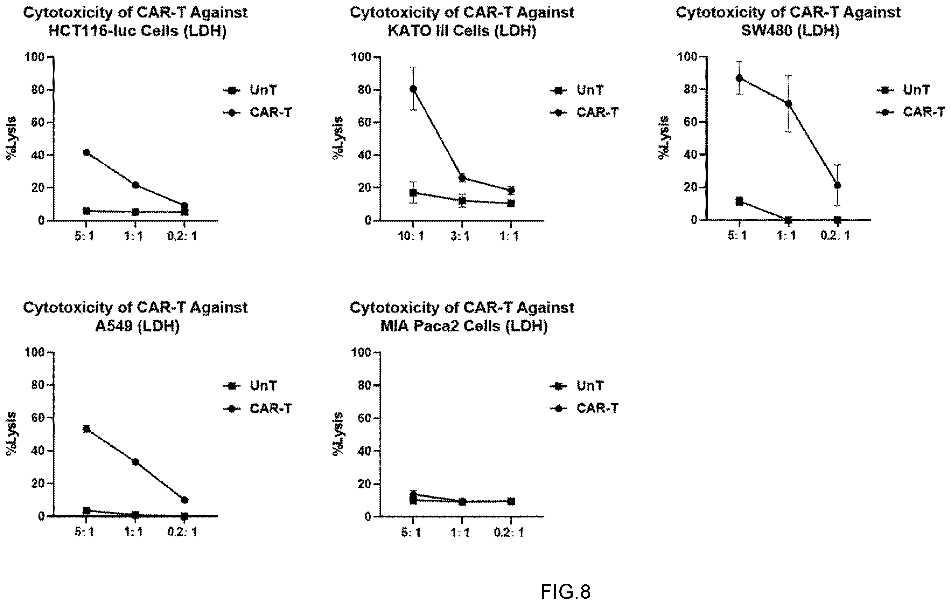

Fig. 8 is a comparison chart of the killing efficiency of CAR-T and reference T cell unT on different tumor cells at different effector/target ratios. Referring to Fig. 8, it can be seen that CAR-T cells specifically kill EpCAM-positive tumor cells HCT116-Luc, SW480, KATO-III, and A549, but do not kill EpCAM-negative tumor cells MIAPaCA-2.

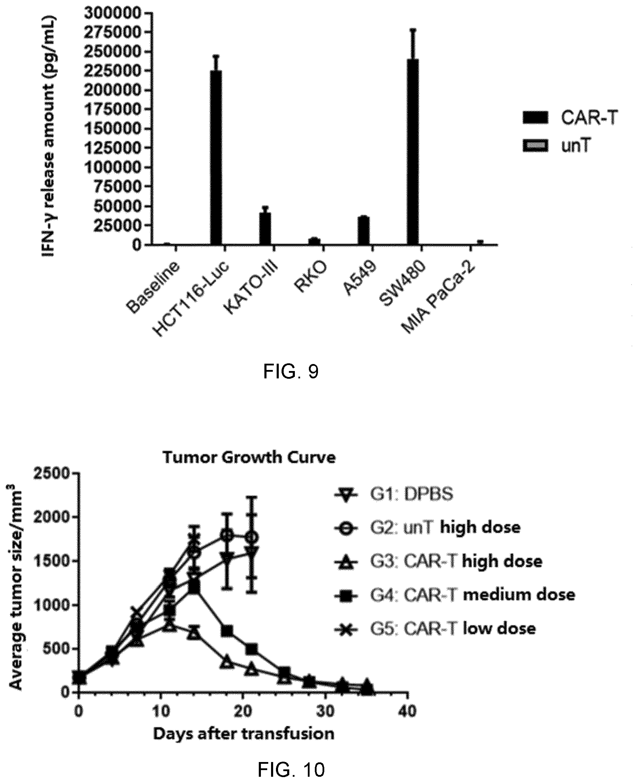

Fig. 9 is a comparison chart of the amount of IFN-γ released after the action of CAR-T and reference T cells (unT) with different tumor cells. Consistent with the results of the killing experiment, referring to Fig. 9, the IFN-γ release of EpCAM CAR-T cells was specifically activated by EpCAM-positive tumor cells HCT116-Luc, SW480, KATO-III, and A549, but not by EpCAM-negative tumor cells MIAPaCA-2. There was no significant activation of IFN-γ release upon co-culture with MIAPaCA-2.

### Example 6. EpCAM-targeting CAR-T Cells Eliminate Tumor Cells in Mice

The example of the present disclosure examined the therapeutic effect and safety of CAR-T on subcutaneous tumor formation of HCT116 in M-NSG mice.

Specifically, M-NSG mice were subcutaneously inoculated with HCT116 to establish the HCT116 tumor model. On day 10 after inoculation, the average tumor volume in mice was approximately 170 mm³. The mice were randomly divided into 5 groups: G1~G5. Each group was administered by tail vein injection with PBS for G1, unT for G2, and 20E6, 2E6, 0.2E6 CAR-T cells for G3, G4, and G5 groups, respectively. Blood samples were collected from the intraocular canthus of the mice in each group on day 1, 8, 15, 22, 28, 36 and 42 after administration, and the persistence of human CD45+ and human CD3+ cells as well as the proportion of CAR+ cells in the peripheral blood of mice were detected by flow cytometry (FACS). The tumor diameter and the body weight of the animals were measured twice a week, the living conditions of the animals were observed, and the abnormal conditions were recorded. During the whole experiment, the mice were euthanized according to the experimental requirements, and the mice were dissected and collected for slice analysis. The number of mice in each group for statistical analysis was 6.

Fig. 10 is a comparison chart of the change in average tumor volume in the mice in each group during the experiment. Referring to Fig. 10, on day 21, the average tumor volume of group G1 was 1586.69 ± 439.96 mm³, and the average tumor volumes of the other groups were 1770.23 ± 460.15 mm³, 272.00 ± 26.42 mm³, 272.00 ± 26.42 mm³, 498.22 ± 47.85 mm³ and 3607.44 ± 510.54 mm³, and the corresponding tumor inhibition rates were -13.11%, 92.97%, 76.99%, and-142.95%. The average tumor volume of each of the groups G3 and G4 was significantly lower than that of the group G1, indicating that CAR-T at the experimental concentrations of 20E6 and 2E6 showed a certain effect of inhibiting tumor growth. On day 42, the tumor tissue of all mice in group G4 was completely eliminated, achieving complete remission.

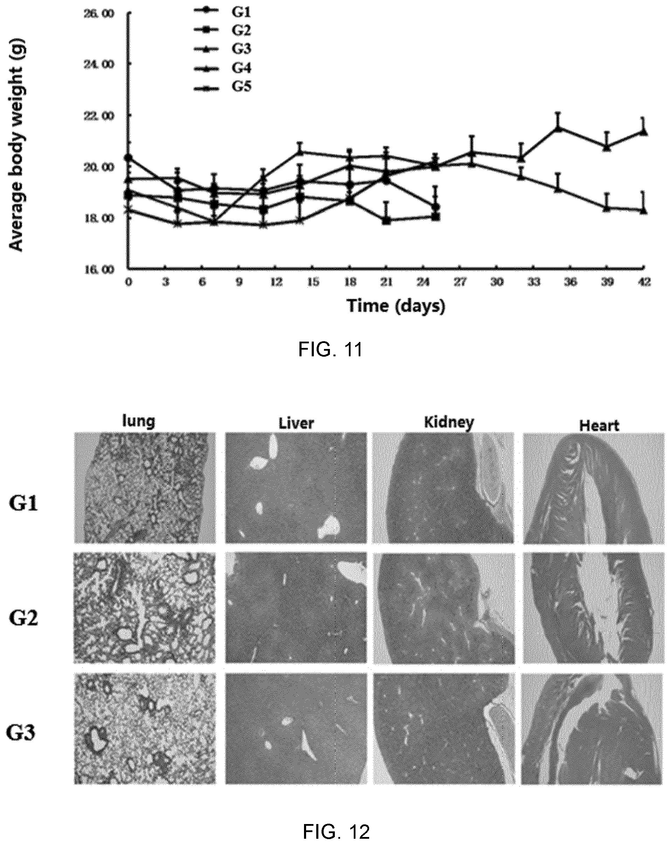

Fig. 11 is a comparison chart of the change in the average body weight of the mice in each group during the experiment. Referring to Fig. 11, on day 21, the weight growth rate of the mice in each group was -4.42%, -5.21%, 6.89%, 1.45% and 7.28%. The mice in groups G3, G4 and G5 could maintain their body weight better during the experiment, indicating that CAR-T did not show obvious toxicity at the three experimental concentrations of 20E6, 2E6, and 0.2E6, while the mice in groups G1 and G2 were not able to maintain body weight well during the experiment.

Further, the mice in groups G1, G2 and G3 were taken for histological anatomical detection, and the representative samples of the formed tissue sections are shown in Fig. 12.

Referring to Fig. 12, the lungs of the mice in group G1 showed moderate tumor cell infiltration or metastasis; the mice in group G2 showed perivascular inflammatory cell infiltration in the lungs, slightly increased neutrophils in the spleen, and slightly inflammatory cell infiltration in the portal area of the liver; the mice in group G3 showed mild perivascular inflammatory cell infiltration in the lungs, but did not show tumor cell infiltration or metastasis, indicating the effect of inhibiting tumor growth. The histopathological changes in G3 tissue sections may be related to the pharmacological effects of the test substance, namely CAR-T, on the tumor-bearing mice, and there was no CAR-T-related toxic response.

Although the embodiments of the present disclosure have been described in detail above, it will be apparent to those skilled in the art that various modifications and changes can be made to these embodiments. However, it should be understood that such modifications and changes are within the scope and spirit of the disclosure as set forth in the appended claims. Furthermore, the disclosure described herein is capable of other embodiments and of being practiced or carried out in various ways.

## Claims

1. A humanized antibody, which comprises a heavy chain variable region and a light chain variable region, wherein:
a sequence of the heavy chain variable region comprises a sequence shown in SEQ ID NO: 1, a sequence shown in SEQ ID NO: 2 and a sequence shown in SEQ ID NO: 3, respectively as a sequence of a first heavy chain complementarity determining region, a sequence of a second heavy chain complementarity determining region and a sequence of a third heavy chain complementarity determining region; and
a sequence of the light chain variable region comprises a sequence shown in SEQ ID NO: 4, a sequence shown in SEQ ID NO: 5 and a sequence shown in SEQ ID NO: 6, respectively as a sequence of a first light chain complementarity determining region, a sequence of a second light chain complementarity determining region and a sequence of a third light chain complementarity determining region.

2. The humanized antibody according to claim 1, wherein,
the sequence of the heavy chain variable region is at least 90% identical to any one of SEQ ID NO: 7, SEQ ID NO: 8, SEQ ID NO: 9, SEQ ID NO: 10 and SEQ ID NO: 11; and
the sequence of the light chain variable region is at least 90% identical to any one of SEQ ID NO: 12, SEQ ID NO: 13, SEQ ID NO: 14 and SEQ ID NO: 15.

3. A chimeric antigen receptor, which comprises an extracellular antigen binding region, a hinge region, a transmembrane region and an intracellular signal region, the extracellular antigen binding region comprising the humanized antibody according to any one of claims 1 to 2, or a fragment of the humanized antibody.

4. The chimeric antigen receptor according to claim 3, wherein a sequence of the hinge region is derived from at least one of CD8α, CD28, 4-1BB, ICOS, OX40, CD40, CD80 and IgG, a sequence of the transmembrane region is derived from at least one of CD2, CD27, LFA-1, CD8α, CD28, 4-1BB, ICOS, OX40, CD40, CD80, CD3ζ and CD3ε, and a sequence of the intracellular signal region is derived from a Toll-like receptor, and at least one of CD2, CD27, LFA-1, CD8a, CD28, 4-1BB, ICOS, OX40, CD40, CD80, DAP10, DAP12, CD3ζ and CD3ε.

5. The chimeric antigen receptor according to claim 3, wherein a sequence of the chimeric antigen receptor is selected from any one of sequences shown in SEQ ID NOs: 18-29.

6. A nucleic acid, which comprises a sequence encoding the humanized antibody according to any one of claims 1 to 2, or a fragment of the sequence.

7. A nucleic acid, which comprises a nucleotide sequence encoding the chimeric antigen receptor according to any one of claims 3 to 5, or a fragment of the sequence.

8. A vector comprising the nucleic acid according to any one of claims 6 to 7.

9. A cell comprising the nucleic acid according to any one of claims 6 to 7 or the vector according to claim 8.

10. Use of a biological product in the manufacture of a reagent for detecting human tumors or a medication for treating human cancers, wherein the biological product is the humanized antibody according to any one of claims 1 to 2, the chimeric antigen receptor according to any one of claims 3 to 5, the nucleic acid according to any one of claims 6 to 7, the vector according to claim 8, or the cell according to claim 9.
